# EUROPEAN PATENT APPLICATION

(11) **EP 0 792 887 A1**
(43) Date of publication of application: **03.09.1997**
(21) Application number: 97105247.7
(22) Date of filing: 15.06.1990
(51) Int. Cl.: C07K 14/765, A61K 38/38, C07K 1/18

(54) **Albumin preparation**

(30) Priority: 15.06.1989 JP 153137/89
(62) Divisional of application: 90909363.5
(71) Applicant: THE GREEN CROSS CORPORATION, Osaka-shi Osaka 541 (JP)
(72) Inventor: Matsuoka, Yasushi, c/o The Green Cross Corp., Hirakata-shi, Osaka 573 (JP); Hase, Shinichiro, c/o The Green Cross Corp., Hirakata-shi, Osaka 573 (JP); Takechi, Kazuo, c/o The Green Cross Corp., Hirakata-shi, Osaka 573 (JP); Tamioka, Shinizi, c/o The Green Cross Corp., Hirakata-shi, Osaka 573 (JP); Yokoyama, Kazumasa, c/o The Green Cross Corp., Hirakata-shi, Osaka 573 (JP)
(74) Representative: Hansen, Bernd, Dr. Dipl.-Chem.

(57) **Abstract**

The present invention has been achieved in order to solve a problem that aggregates, which are considered to be caused by the aggregation of albumin, and contaminating proteins such as transferrin remain in an albumin preparation. Thus the present invention provides an albumin preparation wherein the content of aggregates determined by gel filtration is lower than the detectable limit and the content of transferrin determined by Mancini's method is lower than the detectable limit. The preparation is produced by a process which comprises treating an aqueous solution containing serum albumin with an anion exchanger, then with a cation exchanger and finally subjecting the solution to heat treatment.

## Description

### Technical Field

The invention relates to an albumin preparation. Specifically, the invention relates to a serum albumin preparation in which the content of aggregates and of contaminating proteins is reduced.

### Background Technology

Serum albumin, the protein found in the highest concentration in serum, functions to maintain the osmotic pressure of blood and to transport nutritive substances or metaboliets bound thereto. A preparation containing the above-mentioned serum albumin is used in the treatment of, for example, hypoalbuminemia caused by the loss of albumin and dysfunction of albumin synthesis and hemorrhagic shock. An albumin preparation in the form of an aqueous solution is heat-treated generally so as to inactivate viruses which might contaminate the preparation. The results of gel filtration analysis of a commercially available albumin preparation produced by the above-mentioned method indicate that aggregates were present in said preparation. The aggregates (which are commonly called "polymers" and thus are hereinafter referred to as so) are observed rarely before the above-mentioned heat treatment. Thus, it can be inferred that albumin becomes aggregated upon the heat treatment possibly under the action of contaminating proteins which are unstable to heat. Since commercially available albumin preparations have been widely and safely used, it is believed that these polymers are harmless to human. However it is preferable to minimize the content of polymers in albumin solutions, since the polymers are heat denaturation products.

Further, albumin contains contaminating proteins such as transferrin, the physicochemical properties of which are relatively similar to those of albumin. Since it is difficult to efficiently separate these contaminating proteins from albumin by conventional methods such as fractionation, there is the problem of contamination of an albumin preparation by these proteins. Discription of the Invention

The present invention is directed to solve the above-described problems. As a result of extensive studies, the present inventors found that high purification of albumin makes it possible to reduce the content of contaminating proteins, including transferrin, and to reduce formation of polymer after heat treatment, thus the present invention was completed. Accordingly, an object of the present invention is to provide an albumin preparation containing reduced content of polymers and contaminating proteins, as well as a process for producing the same.

The albumin preparation of the present invention, which has been conceived in order to solve the above-described problems, is characterized by a polymer content determined by gel filtration analysis that is lower than the detectable limit and by a transferrin content determined by Mancini's method that is lower than the detectable limit. The process for producing the albumin preparation of the present invention is characterized by treating an aqueous solution containing serum albumin first with an anion exchanger and second with a cation exchanger and followed by heat treatment. As the anion exchanger and cation exchanger to be used in the treatment of the albumin-containing solution in the above production process, a strongly basic anion exchanger and a strongly acidic cation exchanger are preferred.

The origin of the albumin, which is the major ingredient of the preparation of the present invention and the starting material in the production process of the present invention, is not particularly restricted. Examples thereof include those originating from mammals such as human, bovine and rabbit. In particular, human albumin may be used. As an example of a starting material from which the albumin is to be prepared, the fraction V obtained by Cohn's cold alcoholic fractionation may be used.

The albumin preparation of the present invention may be obtained by dissolving the above-described serum albumin fraction V in an appropriate purified water and treating the albumin-containing aqueous solution thus obtained with an anion exchanger and then with a cation exchanger followed by heat treatment.

In the above-described process, the albumin content of the albumin-containing aqueous solution may be adjusted usually to approximately 0.1 to 30 % (W/V, the same will apply hereinafter unless otherwise noted) and preferably to approximately 1 to 10 %.

According to the present invention, the aqueous albumin solution is first purified by treating with an anion exchanger. Contaminating proteins having an isoelectric point lower than that of albumin, such as haptoglobin and α₁-acidic glycoprotein are removed. As the usable anion exchanger, any inert carrier may be used so long as it has an anion exchange group (for example, a diethylaminoethyl group). Specific examples thereof include anion exchangers commonly used in the art, for example, DEAE-Sepharose® and Q Sepharose® (each manufactured by Pharmacia), DEAE-Toyopearl® and QAE-Toyopearl® (each manufactured by Tosoh Corporation) and A200 Cellulofine® (manufactured by Seikagaku Corporation), anion exchange resins. From the viewpoint of the efficiency of the removal of contaminating proteins, it is preferable to use a strongly basic anion exchanger such as Q-Sepharose or QAE-Toyopearl.

The above treatment with an anion exchanger may be performed by contacting the aqueous albumin solution with the anion exchanger. The amount of the anion exchanger may be appropriately adjusted, depending on the content of the contaminating proteins in the albumin-containing aqueous solution and the performance of the anion exchanger, to 0.1 to 5 ml, usually 3 ml, of the anion exchanger per gram of albumin. This treatment may be performed either by a column method or by a batch method. The column method is preferred from the viewpoint of the efficiency of removal of the contaminating proteins.

When the treatment is to be performed by the column method, the pH value of the above-mentioned aqueous albumin solution is adjusted to 3 to 6, preferably 4.5 to 5.5, and the salt concentration thereof is adjusted to 0.001 to 0.2 M, in terms of sodium chloride, preferably to 0.001 to 0.05 M. Then the resulting aqueous solution is passed through an anion exchanger column, which has been equilibrated with a buffer solution [for example, a 0.02 M sodium acetate buffer (pH 5.1)] and then developed with said buffer solution to recover the unadsorbed fraction. To suppress the denaturation of albumin, the above procedure is preferably performed at a low temperature (usually 10 °C or below).

When the treatment is to be performed by the batch method, an anion exchanger is added to the aqueous albumin solution, which has been adjusted to the conditions as specified above, to thereby contact these materials. After stirring at a temperature of 10 °C or below for 30 minutes to 2 hours, the aqueous solution is separated from the anion exchanger by, for example, centrifugation and the supernatant is recovered.

The pH value and concentration of the aqueous albumin solution thus purified by the above anion exchanger treatment is adjusted, if required. Then, it is further purified by treating with a cation exchanger. This cation exchanger treatment makes it possible to remove contaminating proteins such as transferrin having an isoelectric point higher than that of albumin. As the cation exchanger, any insouble carrier may be used so long as it has a cation exchange group (for example, a sulfo group or a carboxyl group). Specific examples thereof include cation exchangers commonly used in the art [for example, SP-Sephadex® (manufactured by Pharmacia) and SP-Toyopearl® and TSKgelSP-5PW® (each manufactured by Tosoh Corporation)]. From the viewpoint of the efficiency of the removal of contaminating proteins, it is preferable to use a strongly acidic cation exchanger such as SP-Sephadex or SP-Toyopearl.

The above treatment with a cation exchanger may be performed by contacting the aqueous albumin solution purified by the above-mentioned anion exchanger treatment with the cation exchanger. The amount of the cation exchanger may be appropriately adjusted depending on the content of the contaminating proteins in the albumin-containing aqueous solution and the performance of the cation exchanger, to 0.1 to 5 ml, usually 2 ml, of the cation exchanger per gram of albumin. This treatment may be performed either by a column method or by a batch method. However, the column method is preferred from the viewpoint of the efficiency of the removal of the contaminating proteins.

When the treatment is to be performed by the column method, the pH value of the above-mentioned aqueous albumin solution is adjusted to 4.5 to 6.5, preferably to 5.5 to 6.0, and the salt concentration thereof is adjusted to 0.001 to 0.2 M, in terms of sodium chloride, preferably to 0.001 to 0.05 M. Then the aqueous solution is passed through a cation exchanger column, which has been equilibrated with a buffer solution [for example, a 0.02 M sodium acetate buffer (pH 5.5)] and then developed with said buffer solution to recover the unadsorbed fraction. To suppress the denaturation of albumin, the above procedure is preferably performed at a low temperature (usually 10 °C or below).

When the treatment is to be performed by the batch method, a cation exchanger is added to the aqueous albumin solution, which has been adjusted to the conditions as specified above, to thereby contact these materials. After stirring at a temperature of 10 °C or below for 30 minutes to 2 hours, the aqueous solution is separated from the cation exchanger by, for example, centrifugation and the supernatant is recovered.

With respect to the content of contaminating proteins contained in the aqueous albumin solution thus purified by the treatments with the anion exchanger and cation exchanger, the amount of haptoglobin, transferrin and α₁-acidic glycoprotein each is lower than the detectable limit.

The concentration of the aqueous albumin solution in which the content of contaminating proteins have been reduced by the above-described treatments with the anion exchanger and the cation exchanger is adjusted to an appropriate level and the resulting solution is formulated into a preparation of a desired form, for example, filled into an ampule, and then subjected to heat treatment. The albumin preparation of the present invention thus is obtained. The above heat treatment, which aims at inactivating viruses which might contaminate the albumin preparation, is performed in the form of an aqueous solution having an albumin concentration of 5 to 30 %, usually 5 % or 20 to 25 %. The treatment may be performed at a temperature for a period of time each sufficient for inactivating the contaminating viruses. For example, it may be performed at 50 to 70 °C, preferably at approximately 60 °C, for 5 to 20 hours, preferably for approximately 10 hours. Upon the above heat treatment, one or more stabilizers for albumin, for example, N-acetyltryptophan sodium or sodium caprylate, may be added, if required. These albumin stabilizers may be used at a rate of from 20 to 60 mg, preferably 40 mg, per gram of the albumin contained in the preparation.

The albumin preparation thus obtained shows a polymer content determined by gel filtration lower than the detectable limit and a transferrin content determined by Mancini's method lower than the detectable limit.

The albumin preparation of the present invention may be used in the same manner and in the same dose as those selected for conventional ones.

### Brief Description of Drawings

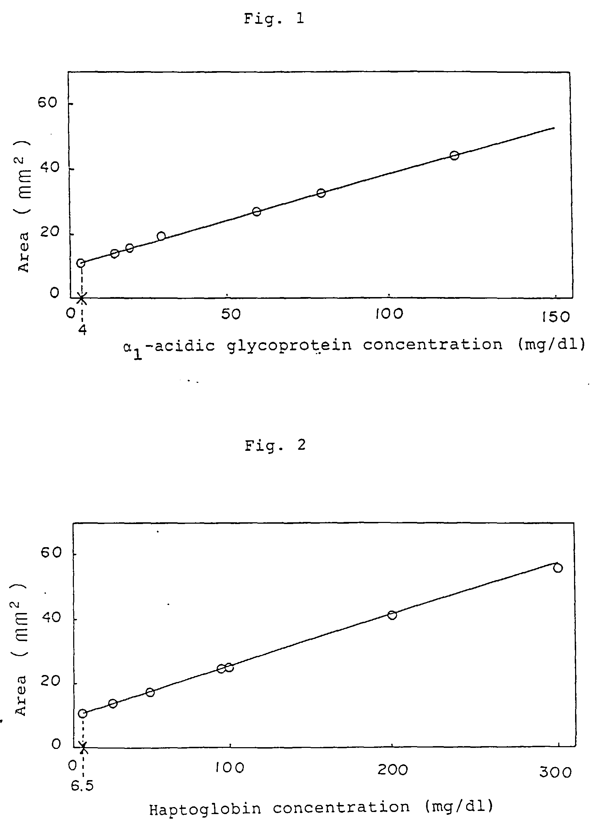

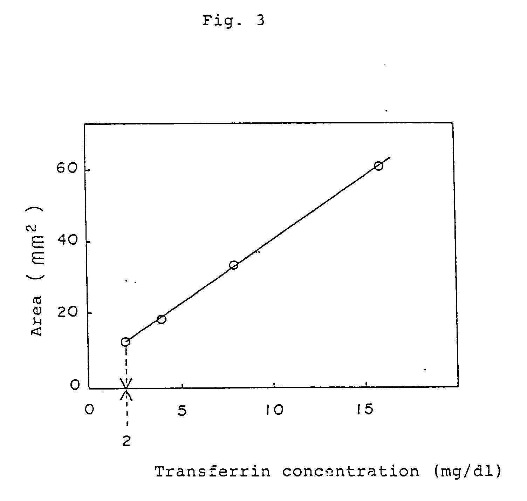

Figs. 1 to 3 are graphs showing standard curves respectively for α₁-acidic glycoprotein, haptoglobin and transferrin each determined by single immunodiffusion.

### Best Mode for Practice of the Invention

The present invention is illustrated further by way of the following non-limiting Examples.

### Example 1

### (1) Preparation of aqueous albumin solution

A paste of the fraction V (500 g) obtained by Cohn's cold alcoholic fractionation was dissolved in 2.0 liters of cold aseptic distilled water and the pH value was adjusted to 4.6 with acetic acid, followed by stirring for approximately 1 hour. Then the solution was filtered (filter: 0.45 µm) at approximately -2 °C and then 2.0 liters of cold aseptic distilled water was further added. The pH value thereof was adjusted to 5.1 with 1 N sodium hydroxide to give an aqueous albumin solution.

### (2) Treatment with anion exchanger

A column (diameter 5 cm x length 18 cm) was packed with QAE- Toyopearl (580 ml) and thoroughly washed with 0.5 M sodium chloride. Then, it was equilibrated with 0.02 M sodium acetate (pH 5.1) to give an anion exchanger column. The aqueous albumin solution obtained in the above treatment (1) was loaded onto the column which was then washed with cold 0.02 M sodium acetate (pH 5.1, 2 liters). The pass-through liquor was combined with the wash liquor and the pH value thereof was adjusted to 5.5 with 0.8 M sodium carbonate.

### (3) Treatment with cation exchanger

A column was packed with SP-Toyopearl (400 ml) and was washed thoroughly with 0.5 M sodium chloride. Then it was equilibrated with 0.02 M sodium acetate (pH 5.5) to give a cation exchanger column. The aqueous albumin solution obtained in the above treatment (2) was passed over the column which was then washed with 0.02 M sodium acetate (pH 5.5, 1.2 liters). The pass-through liquor was combined with the wash liquor. The mixture was dialyzed and then concentrated with Pellicon to achieve an absorbance A₂₈₀ of 149 (albumin concentration: 28 %).

### (4) Heat treatment

To the aqueous albumin solution obtained in the above treatment (3), was added an aqueous solution of stabilizers (containing 5.55 g of N-acetyltryptophan and 3.89 g of sodium caprylate in 100 ml of said solution) at a ratio of 1.2 ml per 10 ml of said aqueous albumin solution. The pH value of the mixture was adjusted to 6.85 with 1 N sodium hydroxide followed by aseptic filtration. Then the albumin concentration was adjusted to 25 % and aliquots of the solution were pipetted into vials. After heat treatment at 60 °C for 10 hours, an albumin preparation was obtained.

When the albumin preparation of the present invention thus obtained was examined for polymer content by gel filtration, no polymer was detected. Another albumin preparation (hereinafter referred to as the comparative preparation) prepared substantially in the same manner as the one described above, except that the SP-Toyopearl treatment step was omitted, showed a polymer content of 2.49 % by weight based on the albumin content. Thus the polymer content in the albumin preparation of the present invention was extremely low. The gel filtration analysis was performed under the following conditions.
(1) Sample: The preparation of the present invention and the comparative preparation were diluted 50-fold with the buffer solution as specified below and filtered (filter: 0.45 µm). Then 20 µl of each solution was poured.
(2) Column: A column (diameter 7.8 mm x length 30 cm) packed with TSKgelG3000SW (manufactured by Tosoh Corporation) was used.
(3) Buffer solution: 0.1 M KH₂PO₄/0.3 M NaCl (pH 6.9).
(4) Flow rate: 1 ml/minute.
(5) Detection wavelength: λ = 280 nm.
(6) Apparatus: Waters HPLC system.

The contents of α₁-acidic glycoprotein (α₁-AG), haptoglobin (Hp) and transferrin (Tf) in the above-obtained preparation of the present invention and the comparative preparation were determined. Table 1 shows the results. The contaminating protein content was determined by the single immunodiffusion method [Mancini's method: Mancini G. et al., Immunochemistry, Vol. 2, No. 3, 235-254 (1965)]. The anti α₁-acidic glycoprotein serum, antihaptoglobin serum and antitransferrin serum employed in the tests were each prepared in rabbits as an immune animal by a conventional method. Figs. 1 to 3 show standard curves of sedimentation area respectively corresponding to the contaminating proteins formed by using single immunodiffusion gels prepared from these antisera.

As shown in Table 1, the contents of α₁-Ag, Hp and Tf in the albumin preparation of the present invention are each lower than the detectable limit, which indicates that the albumin preparation contains little contaminating protein. As Figs. 1 to 3 clearly show, the detectable limit of α₁-AG is 4 mg/dl, that of Hp is 6.5 mg/dl and that of Tf is 2 mg/dl.

**Table 1**

| | Content of contaminating protein (mg/dl) | | |
|---|---|---|---|
| | AG | Hp | Tf |
| Invention preparation | < DL | < DL | < DL |
| Comparative preparation | < DL | < DL | 8.3 |

In the above Table 1, "< DL" means lower than the detectable limit.

### Example 2

An albumin preparation was prepared in the same manner as in Example 1 except that the pH value was adjusted to 5.25 with 0.8 M sodium carbonate in the anion exchanger treatment step (2) and that the pH value was adjusted to 5.25 in the cation exchanger treatment step (3).

The polymer content and contaminating protein contents of the albumin preparation thus obtained were examined in the same manner as in Example 1. As a result, the polymer content was lower than the detectable limit and the Hp, α₁-AG and Tf contents were lower than the detectable limit.

### Industrial Applicability

The albumin preparation of the present invention is excellent in safety and stability as viruses are inactivated by heat treatment, polymer content is low and content of contaminating proteins, such as transferrin, is extremely low.

The process for producing the albumin preparation of the present invention comprises removing contaminating proteins having various isoelectric points from an aqueous albumin solution by treating the solution with an anion exchanger and a cation exchanger and subjecting the solution to heat treatment. According to the process of the present invention, the polymerization caused by the contaminating proteins can be suppressed and an albumin preparation in which the content of polymers and contaminating proteins is reduced and viruses which might contaminate the preparation are inactivated is obtained.

## Claims

1. An albumin preparation whose polymer/aggregate content determined by gel filtration is below the detectable limit, and whose contents of transferrin, haptoglobin and α₁-acidic glycoprotein determined by applying Mancini's method to a 25% W/V aqueous solution of the albumin preparation are respectively less than 2mg/dl, less than 6.5 mg/dl and less than 4 mg/dl.

2. An albumin preparation according to Claim 1 obtainable by a process which comprises the steps of:
(i) treating an aqueous solution containing serum albumin with an anion exchanger at a pH of 5.1 - 5.5 and recovering the unadsorbed fraction;
(ii) treating the unadsorbed fraction obtained from step (i) with a cation exchanger at a pH of 4.5 - 6.5 and recovering the unadsorbed fraction; and
(iii) heat treating the unadsorbed fraction obtained from step (ii).

3. An albumin preparation according to Claim 2, wherein the anion exchange treatment is carried out at a pH of 5.1-5.25.

4. A process for producing an albumin preparation according to Claim 2, wherein a strongly basic anion exchanger is used in the anion exchange treatment.
